(19) Europäisches Patentamt
European Patent Office
Office européen des brevets

(11) **EP 4 450 056 A1**

(12) **DEMANDE DE BREVET EUROPEEN**

(43) Date de publication:
**23.10.2024 Bulletin 2024/43**

(21) Numéro de dépôt: **23305596.1**

(22) Date de dépôt: **18.04.2023**

(51) Classification Internationale des Brevets (IPC):
*A61K 8/73* (2006.01)      *A61K 8/88* (2006.01)
*A61K 8/9728* (2017.01)      *A61Q 1/02* (2006.01)
*A61Q 1/12* (2006.01)      *A61Q 19/00* (2006.01)
*A61K 8/85* (2006.01)

(52) Classification Coopérative des Brevets (CPC):
**A61K 8/88; A61K 8/736; A61K 8/9728; A61Q 1/02;
A61Q 19/00;** A61K 2800/43

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(71) Demandeur: **L'OREAL**
**75008 Paris (FR)**

(72) Inventeurs:
• **BOILEAU, Nathalie**
**94550 Chevilly Larue (FR)**
• **KUSINA, Christophe**
**94550 Chevilly Larue (FR)**
• **SABATIE, Laurent**
**94550 Chevilly Larue (FR)**

(74) Mandataire: **Lavoix**
**2, place d'Estienne d'Orves**
**75441 Paris Cedex 09 (FR)**

(54) **COMPOSITION À BASE DE CHITOSAN ET DE POLYESTER AMIDE À TERMINAISON ESTER**

(57) La présente invention concerne une composition cosmétique comprenant, dans un milieu physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids,

b) au moins un polyester amide à terminaison ester, et
c) au moins un pigment.

Elle se rapporte également à un procédé de maquillage et/ou de soin de la peau et/ou des phanères, dans lequel on applique la composition selon l'invention sur la peau et/ou les phanères.

**Description**

La présente invention concerne une composition cosmétique comprenant, dans un milieu physiologiquement acceptable :

a) au moins 0,01% en poids par rapport au poids total de composition de chitosan natif ayant un poids moléculaire strictement supérieur à 3000 Daltons, cette quantité étant strictement inférieure à 15% en poids,
b) au moins un polyester amide à terminaison ester, et
c) au moins un pigment.

Elle se rapporte également à un procédé de maquillage et/ou de soin de la peau et/ou des phanères, dans lequel on applique la composition selon l'invention sur la peau et/ou les phanères.

Il existe de nombreuses compositions cosmétiques pour lesquelles des propriétés de tenue du film déposé, après application sur les matières kératiniques, sont souhaitées. On peut citer par exemple les rouges à lèvres ou les vernis à ongles. Afin d'obtenir un tel résultat, il est possible d'associer des matières premières particulières, notamment des agents filmogènes. Cependant, la présence de tels agents pour la tenue du film sur les matières kératiniques peut conduire à des compositions desséchantes.

Par ailleurs, on cherche souvent à obtenir des compositions couvrantes.

Le formulateur est donc à la recherche de matières premières et/ou de systèmes permettant d'obtenir des compositions colorées fluides (i.e. qui s'écoulent) dont le dépôt est couvrant, homogène et résistant (i.e. présentant une bonne tenue), en particulier présentant une bonne tenue à l'eau.

Par ailleurs, la formulation de produits cosmétiques respectueux de l'environnement, c'est-à-dire dont la conception et le développement tiennent compte des enjeux environnementaux, devient une préoccupation majeure pour contribuer à relever les défis planétaires.

Il se révèle donc essentiel de proposer des compositions et/ou des procédés de préparation et/ou des ingrédients plus durables permettant ainsi de répondre à ces enjeux environnementaux.

Dans ce contexte, il est important de développer de nouvelles compositions cosmétiques avec une meilleure empreinte carbone notamment en favorisant l'emploi de matières premières renouvelables et/ou avec un bon index de naturalité et/ou d'origine naturelle et plus particulièrement d'origine végétale tout en réduisant l'utilisation de composés d'origine pétrochimique.

La présente invention a pour but de proposer des compositions cosmétiques colorées présentant de bonnes propriétés maquillantes, notamment en termes de couvrance et d'homogénéité, mais aussi ayant une bonne tenue, en particulier ayant une bonne tenue/résistance à l'eau.

Après application, ces compositions laissent un dépôt filmogène couvrant et homogène, qui a une bonne tenue à l'usure. Les films colorés formés sont adhésifs et cohésifs, et présentent une résistance améliorée à l'eau.

Ces compositions comprennent également des ingrédients durables, permettant ainsi de répondre aux enjeux environnementaux.

La présente invention a donc pour objet une composition cosmétique, notamment de maquillage et/ou de soin de la peau et/ou des lèvres, en particulier des lèvres, comprenant, dans un milieu physiologiquement acceptable :

a) au moins 0,01% en poids par rapport au poids total de composition de chitosan natif ayant un poids moléculaire strictement supérieur à 3000 Daltons, cette quantité étant strictement inférieure à 15% en poids,
b) au moins un polyester amide à terminaison ester, et
c) au moins un pigment.

Par « physiologiquement acceptable », on entend un milieu compatible avec les matières kératiniques.

Elle a également pour objet un procédé de maquillage et/ou de soin de la peau et/ou des phanères, dans lequel on applique la composition selon l'invention sur la peau et/ou les phanères.

**Chitosan**

La composition selon l'invention comprend au moins 0,01% en poids par rapport au poids total de la composition d'au moins un chitosan natif ayant un poids moléculaire strictement supérieur à 3000 Daltons (3 kDa).

La quantité de chitosan natif est également strictement inférieure à 15% en poids par rapport au poids total de la composition.

De préférence, le chitosan natif a un poids moléculaire supérieur ou égal à 10 kDa, de préférence supérieur ou égal à 15 kDa, de préférence supérieur ou égal à 20 kDa. De préférence, le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

Le chitosan (ou chitosane) est très peu répandu dans la nature. Il n'est signalé que dans les exosquelettes de certains insectes comme les reines des termites et dans les parois cellulaires d'une classe particulière de champignons, les zygomycètes.

Le chitosan est obtenu par désacétylation de la chitine. La chitine est un polysaccharide composé de plusieurs unités N-acétyl-D-glucosamine reliées entre elles par une liaison de type β (1,4).

La structure chimique idéale du chitosane est un enchaînement de monomères β-D-glucosamine reliés par une liaison glycosidique (1→4).

Par « chitosan » selon l'invention, on entend tout copolymère formé d'unités constitutives N-acétyl-D-glucosamine et D-glucosamine, dont le degré d'acétylation est inférieur à 90%. Le chitosan est constitué des unités sucres glucosamine (unités désacétylées) et d'unités N-acétyl-D-glucosamine (unités acétylées) reliées entre elles par des liaisons de type β (1,4) et constitue un polymère du type Poly(N-acetyl-D-glucosamine)-poly(D-glucosamine).

De préférence, le degré d'acétylation du chitosan est inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

Le degré d'acétylation est le pourcentage d'unités acétylées par rapport au nombre d'unités totales, il peut être déterminé par spectroscopie infrarouge à transformée de Fourier (IR-TF) ou par un titrage par une base forte.

Le chitosan de l'invention est de préférence un polysaccharide préparé à partir d'une origine fongique. Il est notamment extrait et purifié à partir de sources fongiques alimentaires ou biotechnologique sûres et abondantes tels que *Agaricus bisporus* ou *Aspergillus niger.*

Le chitosan de l'invention est de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus.* De préférence le champignon est *Aspergillus niger.*

Le chitosan peut être d'origine OGM, mais de préférence est d'origine non OGM.

Le chitosan selon l'invention est natif, c'est-à-dire qu'il n'est pas modifié. Il ne contient notamment pas de modification chimique.

Une méthode de préparation du chitosan est celle décrite dans la demande WO03068824.

De préférence, le chitosan utilisé dans l'invention est sous forme de poudre. Il est notamment commercialisé par Kitozyme sous le nom Kiosmetine ou Kionutrime.

Le chitosan est de préférence présent en une quantité allant de 0,01% à 14% en poids, de préférence de 0,1 % à 14% en poids, de préférence de 0,1% à 12% en poids, de préférence de 0,2% à 7% en poids, de préférence de 0,25% à 5% en poids par rapport au poids total de la composition.

## Polyester amide à terminaison ester

La composition selon l'invention comprend au moins un polyester amide à terminaison ester (ETPEA).

Le polymère poly(ester-amide) à terminaison ester (ETPEA) conforme à l'invention se présente de préférence sous la forme d'une résine préparée en faisant réagir un diacide, une diamine, un polyol et un monoalcool dans laquelle :

(i) au moins 50 équivalent % dudit diacide comprend un acide gras polymérisé et
(ii) au moins 50 équivalent % de ladite diamine comprend de l'éthylènediamine.

Plus préférentiellement, la composition de résine est telle que (iii) 10 à 60 équivalent % par rapport à la totalité des équivalents en hydroxyle et en amine provenant de la diamine, du polyol et du monoalcool proviennent du monoalcool, (iv) au plus 50 équivalent % par rapport à la totalité des équivalents en hydroxyle et en amine provenant de la diamine, du polyol et du monoalcool proviennent du polyol.

Les polymères de poly(ester-amide) à terminaison ester (ETPEA) conformes à l'invention peuvent être préparés selon le procédé décrit dans le brevet US 6552160.

Le diacide est en général une molécule organique contenant deux groupes acides carboxyliques ou des groupes réactifs équivalents. De manière préférentielle, le diacide est un acide gras polymérisé.

L'acide gras polymérisé est typiquement un mélange comprenant un dimère acide et un trimère acide où chaque dimère peut être saturé, insaturé, cyclique ou acyclique. L'acide gras polymérisé utilisé pour la synthèse du polymère poly(ester-amide) à terminaison ester (ETPEA) est de préférence un dimère acide.

L'acide gras polymérisé est en général formé par chauffage d'acides gras insaturés à longue chaîne par exemple des monoacides carboxyliques en C18 à des températures de l'ordre de 200-250°C en présence d'une argile catalyseur pour polymériser les acides gras. Le produit obtenu comprend en général un dimère acide en particulier un diacide carboxylique en C36 formé par dimérisation de l'acide gras et un trimère acide en particulier un triacide carboxylique en C54 obtenu par trimérisation de l'acide carboxylique. De plus amples détails sur la polymérisation des acides gras sont indiqués en particulier dans le brevet US 3,157,681 et l'ouvrage "Naval Stores-Production, Chemistry and Utilization, D.F. Zinkel and J. Russell (eds.), Pulp. Chem. Assoc. Inc., 1989, Chapitre 23 ».

De manière préférentielle, l'acide gras polymérisé contient moins de 20% en poids de trimère acide et au moins 80% en poids de dimère acide par rapport au poids total de l'acide gras polymérisé. Plus particulièrement, le dimère acide constitue essentiellement la totalité de l'acide gras polymérisé.

Parmi les acides gras insaturés utilisés pour former l'acide gras polymérisé, on peut citer l'acide oléique, l'acide linoléique, l'acide linolénique. On utilise de préférence, des huiles d'acide gras de longue chaîne qui sont des mélanges d'acides insaturés de longue chaîne obtenus par un procédé de réduction en pulpe du bois. On peut également utiliser d'autres sources comme les graines de soja ou le canola. L'acide gras polymérisé utilisable selon l'invention a un indice d'acide de l'ordre de 180 à 200.

L'acide gras polymérisé peut être hydrogéné avant d'être utilisé dans la réaction de formation de la résine. L'hydrogénation permet d'obtenir un point de fusion de la résine légèrement plus élevé ainsi qu'une plus grande stabilité à l'oxydation et de la couleur dans le cas d'une résine légèrement colorée.

Parmi les acides gras polymérisés et notamment les formes hydrogénées disponibles dans le commerce, on peut citer le produit vendu sous la marque UNIDYME par la société ARIZONA CHEMICAL, le produit vendu sous la marque PRIPOL 1015 par la société UNIQEMA, le produit vendu sous la marque EMPOL 1008 par la société COGNIS.

On utilisera plus particulièrement comme acide gras polymérisé un dimère de l'acide linoléique hydrogéné en C36.

En plus de l'acide gras polymérisé ou des équivalents réactifs, le diacide peut comprendre un co-diacide de formule HOOC-R1-COOH où R1 est un composé hydrocarboné en C4-C19, de préférence C4-C12 et plus préférentiellement C4-C8. Les atomes de carbone peuvent s'arranger sous forme linéaire, ramifiée ou cyclique et une insaturation peut être présente entre deux atomes adjacents. R1 peut être aliphatique or aromatique.

Le réactif diamine possède deux groupes amines qui sont de préférence des amines primaires et représentés par la formule HN(R2a)-R2-N(R2a)H dans laquelle R2a désigne hydrogène, un groupe alkyle ou bien forme avec R2 ou un autre radical R2a un hétérocycle.

On utilisera plus particulièrement comme diamine l'éthylène diamine, i.e. R2a est hydrogène et R2 est -CH2-CH2-.

Les diamines autres que l'éthylènediamine seront désignés dans le texte comme co-diamines. Lorsqu'elles sont présentes, les co-diamines sont utilisées à de faibles quantités par rapport à l'éthylènediamine.

Le monoalcool peut être représenté par la formule R3-OH où R3 est de préférence un groupe hydrocarboné ayant au moins 10 atomes de carbone. Ainsi le monoalcool peut être décrit comme un alcool monohydrique.

Selon une forme particulière R3 est un groupe hydrocarboné en C10-C30, préférentiellement un groupe hydrocarboné en C12-C24 et encore plus particulièrement un radical hydrocarboné en C18. On entend, au sens de l'invention par groupe hydrocarboné en C10-C30, tout groupe ayant au moins 10 atomes de carbone mais au plus 30 atomes de carbone. Les atomes de carbone peuvent être arrangés de manière linéaire, ramifié ou cyclique et le radical hydrocarboné peut être saturé ou insaturé.

Selon une forme particulièrement préférée, R3 est linéaire et le groupe hydroxyle est situé sur un carboné terminal, i.e. le monoalcool est primaire. Parmi les monoalcools pouvant être utilisés pour préparer la résine ETPEA, on peut citer dodécanol-1, tétradecanol-1, hexadécanol-1 (alcool cétylique), octadécanol-1 (alcool stéarylique), eicosanol-1 (alcool arachidylique) et docosanol-1 (alcool béhénylique).

Le monoalcool réactif peut contenir un groupe alkylène, i.e. un groupe alkyle une insaturation entre deux atomes de carbone adjacents.

Un autre monoalcool réactif utilisable selon l'invention peut être un alcool de Guerbet de formule H-C(Ra)(Rb)-CH2-OH où Ra et Rb, identiques ou différents, désignent, de préférence, un groupe hydrocarboné en C6-C20. Les alcools de Guerbet sont notamment décrits dans l'ouvrage Dictionary For Auxiliaries For Pharmacy, Cosmetics And Related Fields, H. P. Fiedler, 3rd Ed., 1989, Cantor Aulendorf. On utilisera plus particulièrement le Hexadecyloctadecanol-2 ayant 34 atomes de carbone.

Un autre type de monoalcool réactif utilisable selon l'invention est une cire alcoolique linéaire. Parmi les cires linéaires alcooliques disponibles sur le marché on peut citer les produits vendus sous la marque UNILIN par la société Petrolite Corporation (Tulsa, Okla.). Ces cires alcooliques linéaires sont en général un mélange d'alcools linéaires ayant au moins 20 atomes de carbone et plus particulièrement au moins 24 atomes de carbone. La technique de l'Osmométrie à Pression de Vapeur (OPV) peut être utilisée pour caractériser le poids moléculaire moyen en nombre d'un mélange d'alcools. Selon une forme particulière, le mélange de cires linéaires monoalcooliques présente un poids moléculaire moyen en nombre mesuré en OPV d'environ 200 à environ 800, de préférence d'environ 300 à environ 600. Un alcool linéaire monohydrique en C22 pur a un poids moléculaire mesuré en OPV de 326.

Conformément à la présente invention, on utilisera de préférence un monoalcool pur ou un mélange de monoalcools linéaires tels que par exemple eicosanol-1 (C20), docosanol-1 (C22, alcool béhénylique), dotriacontanol (C32), tétratriacontanol (C34), pentatriacontanol (C35), tétracontanol (C40), tétraacontanol (C44), dopentaacontanol (C54), tétrahexaacontanol (C64), dohéxaacontanol (C72).

On utilisera plus particulièrement l'octadécanol-1 appelé plus communément alcool stéarylique.

Un dernier ingrédient nécessaire à la préparation de la résine d'ETPEA est un polyol ou alcool polyhydrique. Le polyol a comme structure R4(OH)n où R4 désigne un groupe organique n-valent. Par exemple R4 peut être un groupe organique en C2-C20 sans substitution hydroxyle. Comme autre exemple, R4 peut être un groupe hydrocarboné, n vaut en général 2, 3, 4, 5 ou 6.

Parmi les polyols utilisables selon l'invention, on peut citer l'éthylène glycol, propylène glycol, butylène glycol, glycérol, triméthylolpropane, pentaérythritol, néopentylglycol, tris(hydroxylméthyl)methanol, di-pentaérythritol, et tripentaérythritol. On utilisera plus particulièrement le néopentylglycol.

Des réactifs équivalents des diacides et/ou des réactifs équivalents aux diamines peuvent être également utilisés pour la préparation de la résine d'ETPEA. Par exemple, des diesters peuvent être utilisés à la place de certains ou de tous les diacides dans la réaction formant la résine d'ETPEA. On entend par diester tout produit d'estérification d'un diacide avec des molécules à fonction hydroxyle. De tels diesters sont de préférence obtenus à partir de molécules relativement volatiles contenant des fonctions hydroxyle afin que lesdites molécules puissent être facilement éliminées du récipient de réaction après la réaction du mono-alcool et/ou de la diamine avec le diester. Un diester inférieur en particulier un produit d'estérification ou diestérification d'un diacide tel que défini précédemment avec un monoalcool en C1-C4 (ie : méthanol, éthanol, propanol et butanol), peut être utilisé à la place de certains ou tous les diacides dans la réaction formant la résine d'ETPEA. Les halogénures d'acide peuvent être également utilisés à la place de certains ou de tous les diacides dans la réaction formant la résine d'ETPEA. De même que le monoalcool peut être estérifié par un diacide volatile, par exemple l'acide acétique, avant d'être utilisé dans la réaction formant la résine d'ETPEA. De tels réactifs équivalents ne sont cependant préférentiels dans la mesure où ils introduisent des groupes réactifs dans le récipient de réaction.

De manière préférentielle, les équivalents en acide carboxylique doivent être substantiellement égaux aux équivalents combinés en hydroxyle apportés par le monoalcool et le polyol et en amine apportés par la diamine. Autrement dit, chacun des indices d'acide et d'amine de la résine conforme à l'invention doit être de préférence inférieur à 25, plus préférentiellement inférieur à 15, et plus particulièrement inférieur à 10, plus particulièrement inférieur à 5.

Lorsqu'on utilise un co-diacide pour préparer la résine d'ETPEA, le co-diacide ne doit pas représenter plus de 50% des équivalents en acide carboxylique dans le mélange réactionnel. Autrement dit, le co-diacide représente de 0 à 50 % équivalents, plus préférentiellement de 0 à 25% et encore plus préférentiellement de 0 à 10% des équivalents en acide dans le mélange réactionnel.

Lorsqu'on utilise une co-diamine pour préparer la résine d'ETPEA le co-diacide ne doit pas représenter plus de 50% des équivalents en acide carboxylique dans le mélange réactionnel. Autrement dit, la co-diamine représente de 0 à 50 % équivalents, plus préférentiellement de 0 à 25% et encore plus préférentiellement de 0 à 10% des équivalents en acide dans le mélange réactionnel.

Les équivalents en hydroxyle provenant du polyol sont de préférence inférieurs ou égaux à 50% par rapport à la totalité des équivalents en hydroxyle et en amine apportés par les réactifs polyol, monoalcool et diamine. Selon un mode particulier de l'invention, les équivalents en hydroxyle provenant du polyol peuvent être inférieurs ou égaux à 40%, ou inférieurs ou égaux à 30% ou encore inférieurs ou égaux à 20% par rapport à la totalité des équivalents en hydroxyle et en amine apportés par les réactifs polyol, monoalcool et diamine.

Les équivalents en amine de préférence varient de 0,3 à 0,75 par rapport à la totalité des équivalents en hydroxyle et en amine apportés par les réactifs polyol, monoalcool et diamine. Selon un mode particulier de l'invention, les équivalents en hydroxyle provenant du polyol varient de 0,05 à 0.45 par rapport à la totalité des équivalents en hydroxyle et en amine apportés par les réactifs polyol, monoalcool et diamine. Selon un mode particulier de l'invention, les équivalents en hydroxyle provenant du monoalcool varient de 0,20 à 0,45 par rapport à la totalité des équivalents en hydroxyle et en amine apportés par les réactifs polyol, monoalcool et diamine.

On utilisera plus particulièrement un polymère poly(ester-amide) à terminaison ester de nom INCI Polyamide-8 qui est un copolymère de diacide linoléique hydrogéné, d'éthylènediamine, de néopentylglycol et d'alcool stéarylique (BIS-STEARYL ETHYLENEDIAMINE/NEOPENTYL GLYCOUSTEARYL HYDROGENATED DIMER DILINOLEATE COPOLYMER). Ce copolymère est notamment vendu sous le nom commercial OLEOCRAFT® LP20 vendu par la société Croda.

Le ou les polymère(s) poly(ester-amide) à terminaison ester est (sont) de préférence présent(s) dans la composition en une teneur allant de 0,1 à 20%, mieux de 0,2 à 15% en poids, de préférence de 0,3 à 12% en poids, de préférence de 0,5 à 10% en poids, de préférence de 0,8 à 8% en poids, de préférence de 1 à 7% en poids par rapport au poids total de ladite composition.

De préférence, le ratio pondéral (chitosan : ETPEA) est d'au moins 0,3, de préférence, au moins 0,4. De préférence, le ratio pondéral (chitosan : ETPEA) est compris entre 0,3 et 5, de préférence compris entre 0,4 et 4, de préférence compris entre 0,5 et 3, de préférence compris entre 0,5 et 2.

**Pigments**

La composition selon l'invention comprend au moins un pigment.

On entend par « pigments » des particules blanches ou colorées, minérales ou organiques, insolubles dans un milieu aqueux, destinées à colorer la composition et/ou le dépôt résultant.

Les pigments peuvent être présents, dans la composition, en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids.

Pigments minéraux

Selon un mode de réalisation particulier, les pigments utilisés selon l'invention sont choisis parmi les pigments minéraux.

Par « pigment minéral », on entend tout pigment qui répond à la définition de l'encyclopédie Ullmann dans le chapitre pigment inorganique. On peut citer, parmi les pigments minéraux utiles dans la présente invention, le dioxyde de titane, les oxydes de fer (noir, jaune ou rouge), les oxydes de zirconium ou de cérium, les oxydes de zinc ou de chrome, le violet de manganèse, le bleu outremer, l'hydrate de chrome et le bleu ferrique, les poudres métalliques comme la poudre d'aluminium et la poudre de cuivre. Les pigments minéraux suivants peuvent aussi être utilisés : $Ta_2O_5$, $Ti_3O_5$, $Ti_2O_3$, $TiO$, $ZrO_2$ en mélange avec $TiO_2$, $ZrO_2$, $Nb_2O_5$, $CeO_2$, $ZnS$.

La taille du pigment utile dans le cadre de la présente invention est en général supérieure à 100 nm et peut aller jusqu'à 10 $\mu$m, de préférence de 200 nm à 5 $\mu$m, et plus préférentiellement de 300 nm à 1 $\mu$m. Selon une forme particulière de l'invention, les pigments présentent une taille caractérisée par un D[50] supérieur à 100 nm et pouvant aller jusqu'à 10 $\mu$m, de préférence de 200 nm à 5$\mu$m, et plus préférentiellement de 300 nm à 1 $\mu$m. Les tailles sont mesurées par diffusion statique de la lumière au moyen d'un granulomètre commercial de type MasterSizer 3000® de chez Malvern, permettant d'appréhender la répartition granulométrique de l'ensemble des particules sur une large gamme pouvant aller de 0,01 $\mu$m à 1000 $\mu$m. Les données sont traitées sur la base de la théorie classique de diffusion de Mie. Cette théorie est la plus adaptée pour des distributions de taille allant du submicronique au multi-micronique, elle permet de déterminer un diamètre « effectif » de particules. Cette théorie est notamment décrite dans l'ouvrage de Van de Hulst, H.C., « Light Scattering by Small Particles », Chapitres 9 et 10, Wiley, New York, 1957. D[50] représente la taille maximale que présente 50 % en volume les particules.

Dans le cadre de la présente invention, les pigments minéraux sont plus particulièrement l'oxyde de fer et/ou le dioxyde de titane.

Comme pigments minéraux utilisables dans l'invention, on peut également citer les nacres. Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques. On peut également citer, à titre d'exemple de nacres, le mica naturel recouvert d'oxyde de titane, d'oxyde de fer, de pigment naturel ou d'oxychlorure de bismuth. Les nacres peuvent plus particulièrement posséder une couleur ou un reflet jaune, rose, rouge, bronze, orangé, brun, or et/ou cuivré.

Parmi les pigments utilisables selon l'invention, on peut également citer ceux à effet optique différent d'un simple effet de teinte conventionnel, c'est-à-dire unifié et stabilisé tel que produit par les matières colorantes classiques, comme par exemple, les pigments monochromatiques.

Au sens de l'invention, « stabilisé » signifie dénué d'effet de variabilité de la couleur avec l'angle d'observation ou encore en réponse à un changement de température. Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Pigments organiques

Selon un autre mode de réalisation de l'invention, la matière colorante pigmentaire est un pigment organique, synthétique, naturel ou d'origine naturelle.

Par « pigment organique », on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, et les pigments obtenus par polymérisation oxydante de dérivés indoliques, phénoliques tels qu'ils sont décrits dans le brevet FR 2 679 771.

Les pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque.

Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090). A titre d'exemples de laques, on peut citer le produit connu sous la dénomination D&C Red 7 (CI 15 850 :1).

De préférence, le pigment de l'invention est choisi parmi les pigments minéraux, de préférence parmi le dioxyde de titane, les oxydes de fer, les oxydes de zirconium ou de cérium, les oxydes de zinc ou de chrome et leurs mélanges. De préférence, le pigment de l'invention est choisi parmi le dioxyde de titane, les oxydes de fer et leurs mélanges.

**Milieu physiologiquement acceptable**

La composition selon l'invention comprend un milieu physiologiquement acceptable.

Ledit milieu comprend de préférence au moins une phase aqueuse et au moins une phase huileuse. De préférence, la phase huileuse est dispersée dans la phase aqueuse ; la composition selon l'invention est alors une émulsion huile-dans-eau (H/E).

Phase aqueuse

La phase aqueuse comprend au moins de l'eau. L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle.

La composition comprend de préférence au moins 5% en poids d'eau par rapport au poids total de la composition, de préférence au moins 10% en poids, de préférence au moins 20% en poids, de préférence au moins 30% en poids, de préférence au moins 40% en poids. La composition comprend de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50%.

La phase aqueuse peut également comprendre au moins un solvant organique miscible dans l'eau à 25°C.

De préférence, le solvant organique miscible dans l'eau est choisi parmi les alcools, les polyols et leurs mélanges.

Parmi les alcools, on peut citer les alcools en $C_1$-$C_{10}$, plus préférentiellement en $C_1$-$C_5$, tels que l'éthanol, l'isopropanol, le propanol et le butanol.

Le polyol est, de préférence, choisi parmi les polyols ayant de 2 à 20 atomes de carbone, plus préférentiellement de 2 à 6 atomes de carbone, comme le glycérol, le diglycérol, le propylèneglycol, l'isoprène glycol, le dipropylèneglycol, le butylène glycol, l'hexylène glycol, le 1,2-propanediol, le 1,3-propanediol, le pentylène glycol, les polyéthylèneglycols ayant de 2 à 200 motifs d'oxyde d'éthylène et leurs mélanges.

La composition peut comprendre de 1% à 25% en poids de solvant organique miscible dans l'eau, par rapport au poids total de la composition, plus préférentiellement de 2% à 20% en poids, encore plus préférentiellement de 3% à 15% en poids.

Phase huileuse

La phase huileuse comprend au moins une huile. Elle peut contenir en outre d'autres corps gras. Cette phase huileuse est structurée par la présence de l'ETPEA.

On entend par huile, tout corps gras sous forme liquide à température ambiante (20 - 25°C) et à pression atmosphérique (760 mm Hg). Une huile convenant à l'invention peut être volatile ou non volatile. Elle peut être siliconée, hydrocarbonée ou fluorée, de préférence hydrocarbonée.

Au sens de la présente invention, on entend par « huile siliconée », une huile comprenant au moins un atome de silicium, et notamment au moins un groupe Si-O. On entend par « huile hydrocarbonée », une huile contenant principalement des atomes d'hydrogène et de carbone. On entend par « huile fluorée », une huile comprenant au moins un atome de fluor.

Une huile hydrocarbonée convenant à l'invention peut être une huile hydrocarbonée animale, une huile hydrocarbonée végétale, ou une huile hydrocarbonée synthétique. Une huile hydrocarbonée convenant à l'invention peut en outre éventuellement comprendre des atomes d'oxygène, d'azote, de soufre et/ou de phosphore, par exemple, sous la forme de groupes hydroxyle, amine, amide, ester, éther ou acide, et en particulier sous la forme de groupes hydroxyle, ester, éther ou acide.

Par "huile volatile", on entend au sens de l'invention une huile susceptible de s'évaporer au contact de la peau ou de la fibre kératinique en moins d'une heure, à température ambiante et pression atmosphérique. Le ou les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Comme huiles hydrocarbonées non volatiles utilisables selon l'invention, on peut notamment citer :

(i) les huiles hydrocarbonées d'origine végétale telles que les triesters de glycérides qui sont en général des triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande et notamment l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat, de noix de coco ; ou encore les triglycérides des acides caprylique/caprique comme ceux vendus par la société Stéarineries Dubois ou ceux vendus sous les dénominations Miglyol 810, 812 et 818 par la société Dynamit Nobel,
(ii) les éthers de synthèse ayant de 10 à 40 atomes de carbone ;
(iii) les hydrocarbures linéaires ou ramifiés, d'origine minérale ou synthétique tels que l'huile de vaseline, les polydécènes, le polyisobutène hydrogéné tel que le parléam, le squalane et leurs mélanges (huiles minérales);
(iv) les esters de synthèse comme les huiles de formule RCOOR' dans laquelle R représente le reste d'un acide gras linéaire ou ramifié comportant de 1 à 40 atomes de carbone et R' représente une chaîne hydrocarbonée notamment ramifiée contenant de 1 à 40 atomes de carbone à condition que R + R' soit > 10, comme par exemple l'huile de Purcellin (octanoate de cétostéaryle), le myristate d'isopropyle, le palmitate d'isopropyle, le benzoate d'alcools en C12-C15 comme le produit vendu sous la dénomination commerciale « Finsolv TN » ou « Witconol TN » par la société WITCO ou « TEGOSOFT TN » par la société EVONIK GOLDSCHMIDT, le benzoate de 2-éthylphenyle comme le produit commercial vendu sous le nom « X-TEND 226 » par la société ISP, le lanolate d'isopropyle, le laurate d'hexyle, l'adipate de diisopropyle, l'isononanoate d'isononyle, l'érucate d'oléyle, le palmitate de 2-éthyl-hexyle, l'isostéarate d'isostéaryle, le sébacate de diisopropyle comme le produit vendu sous la dénomination de « Dub Dis » par la société Stearinerie Dubois, des octanoates, décanoates ou ricinoléates d'alcools ou de polyalcools comme le dioctanoate de propylène glycol ; les esters hydroxyles comme le lactate d'isostéaryle, le malate de di-isostéaryle ; et les esters du pentaérythritol ; les citrates ou tartrates comme les tartrates de di-alkyle linéaire en C12-C13 tels que ceux vendus sous le nom COSMACOL ETI par la Société ENICHEM AUGUSTA INDUSTRIALE ainsi que les tartrates de di-alkyle linéaire en C14-C15 tels que ceux vendus sous le nom COSMACOL ETL par la même société ; les acétates ;

(y) les alcools gras liquides à température ambiante à chaîne carbonée ramifiée et/ou insaturée ayant de 12 à 26 atomes de carbone comme l'octyldodécanol, l'alcool isostéarylique, l'alcool oléique, le 2-hexyldécanol, le 2-butyloctanol, le 2-undécylpentadécanol ;

(vi) les acides gras supérieurs tels que l'acide oléique, l'acide linoléique, l'acide linolénique ;

(vii) les carbonates comme le dicaprylyl carbonate comme le produit vendu sous la dénomination « Cetiol CC » par la société Cognis ;

(viii) les amides grasses comme l'isopropyl N-lauroyl sarcosinate comme le produit vendu sous le nom commercial Eldew SL 205 de chez Ajinomoto et leurs mélanges.

Comme huiles hydrocarbonées volatiles utilisables selon l'invention, on peut notamment citer les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane les huiles vendues sous les noms commerciaux d'Isopars ou de Permetyls, les esters ramifiés en C8-C16, le néopentanoate d'iso-hexyle, et leurs mélanges. On peut citer aussi les alcanes décrits dans les demandes de brevets de la société Cognis WO2007/068371 ou WO2008/155059 (mélanges d'alcanes distincts et différant d'au moins un carbone). Ces alcanes sont obtenus à partir d'alcools gras, eux-mêmes obtenus à partir d'huile de coprah ou de palme. On peut citer les mélanges de n-undécane (C11) et de n-tridécane (C13) obtenus aux exemples 1 et 2 de la demande WO2008/155059 de la Société Cognis. On peut également citer le n-dodécane (C12) et le n-tétradécane (C14) vendus par Sasol respectivement sous les références PARAFOL 12-97 et PARAFOL 14-97, ainsi que leurs mélanges.

De préférence, la composition comprend de 1% à 25% en poids de phase huileuse par rapport au poids total de la composition, plus préférentiellement de 2% à 20% en poids, encore plus préférentiellement de 3% à 15% en poids.

Tensioactifs

La composition peut comprendre au moins un tensioactif. Le tensioactif est de préférence choisi parmi les tensioactifs anioniques, cationiques, non-ioniques, zwitterioniques et amphotères. De préférence, il est choisi parmi :

a) les tensioactifs non ioniques, en particulier de HLB supérieure ou égale à 8 à 25°C, utilisés seuls ou en mélange. On peut citer notamment les esters d'acide gras et de polyglycérol, les esters d'acide gras et de polyéthylène glycol, les alkylC8-C30(poly)glycosides ou leurs mélanges. En particulier, le tensioactif non ionique peut être un ester d'acide gras comprenant de 10 à 18 atomes de carbone et de polyglycérol comprenant de 2 à 10 motifs de glycérol. Dans un mode de réalisation préféré de l'invention, l'ester d'acide gras comprend 12 atomes de carbone et 10 motifs de glycérol, et est le monolaurate de polyglycéryle comprenant 10 motifs de glycérol, i.e. le monolaurate de polyglycéryl-10 (disponible sous la dénomination commerciale DERMOFEEL G 10 L par la société Dr Straetmans). Il peut également être le monooléate de polyglycéryl-2 (disponible sous la dénomination commerciale SUNSOFT Q-17D(G)-C par la société TAIYO KAGAKU), ou bien le monocaprate de polyglycéryl-4 ou caprate de PG-4 (disponible sous la dénomination commerciale TEGOSOFT PC 41 par la société EVONIK GOLDSCHMIDT).

b) les tensioactifs anioniques tels que :

les sels d'acides gras polyoxyéthylénés notamment ceux dérivant des sels alcalins, et leurs mélanges ;
les esters phosphoriques et leurs sels tels que le "DEA oleth-10 phosphate" (Crodafos N 10N de la société CRODA) ou le phosphate de monocétyle monopotassique (Amphisol K de Givaudan) ;
les sulfosuccinates tels que le "Disodium PEG-5 citrate lauryl sulfosuccinate" et le "Disodium ricinoleamido MEA sulfosuccinate" ;
les alkyléthersulfates tels que le lauryl éther sulfate de sodium ;
les iséthionates ;
les acylglutamates tels que le "Disodium hydrogenated tallow glutamate" (AMISOFT HS-21 R® commercialisé par la société AJINOMOTO) et le sodium stearoyl glutamate (AMISOFT HS-11 PF® commercialisé par la société AJINOMOTO) et leurs mélanges ;
les dérivés de soja comme le potassium soyate ;
les citrates, comme le Glyceryl stéarate citrate (Axol C 62 Pellets de Degussa) ;
les dérivés de proline, comme le Sodium palmitoyl proline (Sepicalm VG de Seppic), ou le Mélange de Sodium palmitoyl sarcosinate, Magnésium palmitoyl glutamate, Palmitic acid et Palmitoyl proline (Sepifeel One de Seppic) ;
les lactylates, comme le Sodium stearoyl lactylate (Akoline SL de Karlshamns AB) ;
les sarcosinates, comme le sodium palmitoyl sarcosinate (Nikkol sarcosinate PN) ou le mélange de Stéaroyl sarcosine et Myristoyl sarcosine 75/25 (Crodasin SM de Croda) ;
les sulfonates, comme le Sodium C14-17 alkyl sec sulfonate (Hostapur SAS 60 de Clariant) ;
les glycinates, comme le sodium cocoyl glycinate (Amilite GCS-12 d'Ajinomoto) ;
les sels d'acides gras en C16-C30 notamment ceux dérivant des amines, comme le stéarate de triéthanolamine et/ou le stéarate d'amino-2-méthyl-2-propane di-ol-1,3 ;

c) les tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL ;

et leurs mélanges.

**pH de la composition**

La composition selon l'invention présente un pH inférieur ou égal à 7, de préférence inférieur ou égal à 6,5, de préférence inférieur ou égal à 6,3. Avantageusement, le pH de la composition est compris entre 3 et 6,3, de préférence compris entre 4 et 6,3.

De préférence, la composition cosmétique selon l'invention comprend au moins une base et/ou au moins un acide. La base et l'acide selon l'invention sont connus et classiquement utilisés dans le domaine cosmétique.

La base et/ou l'acide sont notamment utilisés pour ajuster le pH final de la composition entre 3 et 6,3.

L'acide peut par exemple être l'acide citrique.

La base peut être choisie parmi les bases minérales comme par exemple les hydroxydes de métaux alcalins, l'hydroxyde de sodium, l'hydroxyde de potassium.

De préférence, la base de la composition est un hydroxyde de métal alcalin, de préférence l'hydroxyde de sodium ou l'hydroxyde de potassium.

La composition selon l'invention peut comprendre au moins une base en une teneur en matière active allant de 0,5 % à 10 % en poids, par rapport au poids total de la composition, notamment de 1 % à 5 % en poids, de préférence allant de 1 % à 4 % en poids.

La composition conforme à l'invention peut être obtenue de manière classique par l'homme du métier.

Les compositions de l'invention peuvent contenir un ou plusieurs des actifs ou excipients habituels dans les domaines cosmétique et dermatologique, tels que des agents hydratants ; des émollients ; des gélifiants, par exemple des gélifiants naturels ; des actifs ; des antioxydants ; des parfums ; des agents filmogènes ; des conservateurs; et leurs mélanges. Les quantités de ces différents excipients sont celles classiquement utilisées dans les domaines considérés. En particulier, ces quantités varient selon le but recherché et peuvent par exemple aller de 0,01% à 20%, et de préférence de 0,1% à 10% en poids du poids total de la composition.

Bien entendu, l'homme de l'art veillera à choisir le ou les éventuels actifs ou excipients ajoutés à la composition selon l'invention telle que les propriétés avantageuses attachées intrinsèquement à la composition conforme à l'invention ne soient pas, ou substantiellement pas, altérées par l'addition envisagée.

Elle a également pour objet un procédé de maquillage et/ou de soin de la peau et/ou des phanères, dans lequel on applique la composition selon l'invention sur la peau et/ou les phanères.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières par rapport au poids total de composition (% p/p), sauf mention contraire.

**Exemples : préparation de compositions selon l'invention et comparaison avec une composition comparative**

Les compositions E1 à E3 selon l'invention, et E comparative (cf Table 1), ont été préparées par le procédé suivant :

Les gels sont préparés au Rayneri à l'aide d'une défloculeuse, la vitesse d'agitation est adaptée afin d'obtenir un vortex minimal.
Le chitosan est dispersé dans l'eau pendant 5 min.
Le Dermosoft 700B (comprenant l'acide lévulinique et le lévulinate de sodium) est ajouté afin d'obtenir une solution dont la valeur du pH est d'environ 5,7. L'agitation est maintenue 30 min.

Le polyamide-8 (quand il est présent) est dispersé au Rayneri à l'aide d'une défloculeuse dans l'isododécane pendant 3h. Puis la dispersion polyamide-8/isododécane obtenue est ajoutée dans la solution de chitosan et agitée 10 min.

Les pigments sont ensuite introduits et l'agitation est maintenue 10 min.

Puis la tenue à l'eau (*in vitro*) est évaluée. Le protocole d'évaluation est le suivant :

1/ Application

- Découper de la supplale noir au format A4,
- Tracer 8 carrés de 5x5 cm à l'aide d'un pochoir,
- Les produits testés sont homogénéisés au SpeedMixer DAC 400 à 2500 tr/min pendant 30 s,
- Déposer 4x10 μl de produit (3 carrés par produit évalué),
- Appliquer en effectuant des petits ronds afin d'étaler le produit sur la surface puis faire 5 aller-retours sur chaque côté puis effectuer 5 aller sur chaque côté afin d'obtenir un échantillon homogène,
- Laisser sécher 20 min.

2/ Mesure du Y

- Faire 5 mesures du Y (D65) par échantillon appliqué à l'aide du colorimètre CM600D de Konica Minolta et du logiciel Spectra Magic Nx,
- Calculer la moyenne, *[Ymoy (T0)]*, l'écart type *[ET(T0)]* et le pourcentage d'erreur [% *ET(T0)]* de chaque échantillon.

3/ Frottement à l'eau

- Placer un coton de frottement sur la partie 0,5 mils (13 μm) d'un étaleur carré BYK 5355,
- Déposer la supplale sur le banc d'étalement et l'étaleur devant l'échantillon à frotter à l'eau,
- Déposer 100 μl d'eau sur le tissu de frottement en 5x20 μl,
- Poser un poids de 900g sur l'étaleur,
- Avancer l'étaleur sur l'échantillon,
- Faire, de nouveau, 5 mesures du Y (D65) et calculer la moyenne, *[Ymoy (Frotté)]*, l'écart type *[ET(F)]* et le pourcentage d'erreur [% *ET(F)]* pour chaque échantillon appliqué et frotté à l'eau.
- Calculer le pourcentage de différence, Δ *restant,* et le pourcentage d'erreur, % *Erreur,* entre les valeurs avant et après frottement à l'eau de chaque échantillon :

[Math 1]

$$\Delta \ restant = [Ymoy \ (F)] \ / \ Ymoy \ (T0)] \ x100$$

[Math 2]

$$\% \ Erreur = \% \ ET(T) + \% \ ET(F)$$

- Enfin, calculer la moyenne des pourcentages de différence, Δmoy restant et la moyenne des pourcentages d'erreur, *Erreur moy* des 3 échantillons.

Les résultats sont dans le tableau 1.

[Table 1]

| Ingrédients (% p/p) | E comparative | E1 invention | E2 invention | E3 invention |
|---|---|---|---|---|
| Chitosan (Kiosmetine CSH de Kitozyme, à 80% en matière active ou m.a.) | 4 (3,2% m.a.) | 4 (3,2% m.a.) | 4 (3,2% m.a.) | 4 (3,2% m.a.) |
| Dermosoft 700B d'Evonik (Acide lévunilique (AL) présent à 40% en m.a., et lévunilate de sodium (LS) présent à 20% en m.a.) | 5,6 (2,24% m.a. AL) (1,12% m.a. LS) | 5,6 (2,24% m.a. AL) (1,12% m.a. LS) | 5,6 (2,24% m.a. AL) (1,12% m.a. LS) | 5,6 (2,24% m.a. AL) (1,12% m.a. LS) |

(suite)

| Ingrédients (% p/p) | E comparative | E1 invention | E2 invention | E3 invention |
|---|---|---|---|---|
| Polyglycéryl-10 laurate | 0,2 | 0,2 | 0,2 | 0,2 |
| Mélange de pigments natifs d'oxydes de fer et de TiO2 (Tarox Iron Oxide R-800HP, BL-100HPL et LL-100HP de Titan Kogyo, et Hombitan FF Pharma de Venator) | 15 | 15 | 15 | 15 |
| Polyamide-8 (SP Oleocraft LP-20 MBAL-PA-(MV) de Croda) | - | 3,2 | 1,6 | 6,4 |
| Isododécane | - | 6,4 | 3,2 | 12,8 |
| Eau | Qsp 100 | Qsp 100 | Qsp 100 | Qsp 100 |
| Ratio pondéral (chitosan : polyamide-8) | Sans polyamide | 1 | 2 | 0,5 |
| Δ restant (%) | 74 | 91 | 91 | 93 |

Les résultats montrent clairement que le polyamide-8 augmente la performance de tenue à l'eau *in vitro.*

**Revendications**

1. Composition cosmétique, notamment de maquillage et/ou de soin de la peau et/ou des lèvres, en particulier des lèvres, comprenant, dans un milieu physiologiquement acceptable :

   a) au moins 0,01% en poids par rapport au poids total de composition de chitosan natif ayant un poids moléculaire strictement supérieur à 3000 Daltons, cette quantité étant strictement inférieure à 15% en poids,
   b) au moins un polyester amide à terminaison ester, et
   c) au moins un pigment.

2. Composition selon la revendication 1, dans laquelle le chitosan natif a un poids moléculaire supérieur ou égal à 10 kDa, de préférence supérieur ou égal à 15 kDa, de préférence supérieur ou égal à 20 kDa, de préférence un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

3. Composition selon la revendication 1 ou 2, dans laquelle le degré d'acétylation du chitosan est inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

4. Composition selon l'une des revendications précédentes, dans laquelle le chitosan natif est extrait et purifié à partir *d'Agaricus bisporus* ou *Aspergillus niger,* de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus,* de préférence le champignon est *Aspergillus niger.*

5. Composition selon l'une des revendications précédentes, dans laquelle le chitosan natif est présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

6. Composition selon l'une des revendications précédentes, dans laquelle le polyester amide à terminaison ester se présente sous la forme d'une résine préparée en faisant réagir un diacide, une diamine, un polyol et un monoalcool dans laquelle :

   (i) au moins 50 équivalent % dudit diacide comprend un acide gras polymérisé et

(ii) au moins 50 équivalent % de ladite diamine comprend de l'éthylènediamine.

7. Composition selon la revendication 6, dans laquelle l'acide gras polymérisé est un dimère de l'acide linoléique hydrogéné en C36 ; et de préférence le diacide comprend un co-diacide de formule HOOC-R1-COOH, où R1 est un composé hydrocarboné en C4-C19, de préférence C4-C12 et plus préférentiellement C4-C8.

8. Composition selon la revendication 6 ou 7, dans laquelle le monoalcool est représenté par la formule R3-OH où R3 est un groupe hydrocarboné ayant au moins 10 atomes de carbone, ou bien un alcool de Guerbet de formule H-C(Ra)(Rb)-CH2-OH où Ra et Rb, identiques ou différents, désignent, un groupe hydrocarboné en C6-C20, de préférence le Hexadecyloctadecanol-2.

9. Composition selon l'une des revendications 6 à 8, dans laquelle le polyol est choisi parmi l'éthylène glycol, propylène glycol, butylène glycol, glycérol, triméthylolpropane, pentaérythritol, néopentylglycol, tris(hydroxylméthyl)methanol, di-pentaérythritol, et tripentaérythritol, plus particulièrement le néopentylglycol.

10. Composition selon l'une des revendications précédentes, dans laquelle le polyester amide à terminaison ester est un copolymère de diacide linoléique hydrogéné, d'éthylènediamine, de néopentylglycol et d'alcool stéarylique.

11. Composition selon l'une des revendications précédentes, dans laquelle le polyester amide à terminaison ester est présent dans la composition en une teneur allant de 0,1 à 20%, mieux de 0,2 à 15% en poids, de préférence de 0,3 à 12% en poids, de préférence de 0,5 à 10% en poids, de préférence de 0,8 à 8% en poids, de préférence de 1 à 7% en poids par rapport au poids total de ladite composition.

12. Composition selon l'une des revendications précédentes, dans laquelle le ratio pondéral (chitosan : ETPEA) est d'au moins 0,3, de préférence, au moins 0,4, de préférence compris entre 0,3 et 5, de préférence compris entre 0,4 et 4, de préférence compris entre 0,5 et 3, de préférence compris entre 0,5 et 2.

13. Composition selon l'une des revendications précédentes, dans laquelle le pigment est présent en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids ; et/ou est choisi parmi les pigments minéraux et les pigments organiques.

14. Composition selon l'une des revendications précédentes, dans laquelle le milieu physiologiquement acceptable comprend au moins une phase aqueuse et au moins une phase huileuse ; de préférence la phase huileuse est dispersée dans la phase aqueuse.

15. Composition selon l'une des revendications précédentes, qui comprend au moins un tensioactif, de préférence choisi parmi les tensioactifs anioniques, cationiques, non-ioniques, zwitterioniques et amphotères.

16. Procédé de maquillage et/ou de soin de la peau et/ou des phanères, dans lequel on applique la composition selon l'une des revendications précédentes sur la peau et/ou les phanères.

Europäisches
Patentamt

European
Patent Office

Office européen
des brevets

# RAPPORT DE RECHERCHE EUROPEENNE

Numéro de la demande

**EP 23 30 5596**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 8 529 921 B2 (FILBRY ALEXANDER [DE]; KROEPKE RAINER [DE] ET AL.) 10 septembre 2013 (2013-09-10) * revendications 1-20; exemples 1-6 * ----- | 1-16 | INV. A61K8/73 A61K8/88 A61K8/9728 A61Q1/02 |
| A | WO 2020/007686 A1 (OREAL [FR]) 9 janvier 2020 (2020-01-09) * revendications 1-17 * * page 29, ligne 20 - page 31, ligne 11 * ----- | 1-16 | A61Q1/12 A61Q19/00 A61K8/85 |
| A | US 2021/121390 A1 (KERGOSIEN GUILLAUME [FR]) 29 avril 2021 (2021-04-29) * page 30; revendications 1-17 * * alinéas [0005], [0347] - [0355] * ----- | 1-16 | |
| A | WO 03/072610 A1 (COGNIS DEUTSCHLAND GMBH [DE]; SUMSER MARKUS [DE] ET AL.) 4 septembre 2003 (2003-09-04) * le document en entier * ----- | 1-16 | |

**DOMAINES TECHNIQUES RECHERCHES (IPC)**

A61K
A61Q

Le présent rapport a été établi pour toutes les revendications

| Lieu de la recherche | Date d'achèvement de la recherche | Examinateur |
|---|---|---|
| Munich | 5 octobre 2023 | Nopper, Agathe |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...............................................................................
& : membre de la même famille, document correspondant

EPO FORM 1503 03.82 (P04C02)

**EP 4 450 056 A1**

## ANNEXE AU RAPPORT DE RECHERCHE EUROPEENNE
## RELATIF A LA DEMANDE DE BREVET EUROPEEN NO.

EP 23 30 5596

05-10-2023

| Document brevet cité au rapport de recherche | | Date de publication | Membre(s) de la famille de brevet(s) | | Date de publication |
|---|---|---|---|---|---|
| US 8529921 | B2 | 10-09-2013 | DE | 102008051007 A1 | 15-04-2010 |
| | | | EP | 2346488 A2 | 27-07-2011 |
| | | | US | 2011268674 A1 | 03-11-2011 |
| | | | WO | 2010043351 A2 | 22-04-2010 |
| WO 2020007686 | A1 | 09-01-2020 | CN | 112437686 A | 02-03-2021 |
| | | | EP | 3817824 A1 | 12-05-2021 |
| | | | KR | 20210025092 A | 08-03-2021 |
| | | | US | 2021361555 A1 | 25-11-2021 |
| | | | WO | 2020007686 A1 | 09-01-2020 |
| US 2021121390 | A1 | 29-04-2021 | CN | 112384285 A | 19-02-2021 |
| | | | EP | 3817823 A1 | 12-05-2021 |
| | | | ES | 2928381 T3 | 17-11-2022 |
| | | | FR | 3083116 A1 | 03-01-2020 |
| | | | KR | 20210025091 A | 08-03-2021 |
| | | | US | 2021121390 A1 | 29-04-2021 |
| | | | WO | 2020007687 A1 | 09-01-2020 |
| WO 03072610 | A1 | 04-09-2003 | DE | 10208550 A1 | 04-09-2003 |
| | | | WO | 03072610 A1 | 04-09-2003 |

EPO FORM P0460

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03068824 A
- US 6552160 B
- US 3157681 A
- FR 2679771
- EP 1184426 A
- WO 2007068371 A
- WO 2008155059 A

**Littérature non-brevet citée dans la description**

- Naval Stores-Production, Chemistry and Utilization. Pulp. Chem. Assoc. Inc, 1989
- **CANTOR AULENDORF.** Dictionary For Auxiliaries For Pharmacy, Cosmetics And Related Fields. 1989
- **VAN DE HULST, H.C.** Light Scattering by Small Particles. Wiley, 1957, 50